# EUROPÄISCHE PATENTSCHRIFT

(11) **EP 0 888 745 B1**
(45) Veröffentlichungstag und Bekanntmachung des Hinweises auf die Patenterteilung: **05.11.2003**
(21) Anmeldenummer: 98202145.3
(22) Anmeldetag: 26.06.1998
(51) Int. Cl.: A61B 6/04, A61B 6/00

(54) **Röntgenuntersuchungsgerät mit einem schwenkbaren Patientenlagerungstisch**
X ray examination apparatus provided with a pivotable patient support
Installation d'examen par rayons X munie d'un support pivotable pour patient

(30) Priorität: 02.07.1997 DE 19728108
(43) Veröffentlichungstag der Anmeldung: 07.01.1999
(73) Patentinhaber: Philips Intellectual Property & Standards GmbH, 20099 Hamburg (DE); Koninklijke Philips Electronics N.V., 5621 BA Eindhoven (NL)
(72) Erfinder: Schwieker, Horst, Röntgenstrasse 24, 22335 Hamburg (DE); Kayser, Harald, Röntgenstrasse 24, 22335 Hamburg (DE); Haarmann, Heinz, Röntgenstrasse 24, 22335 Hamburg (DE); Rothenstein, Jürgen, Röntgenstrasse 24, 22335 Hamburg (DE); Csikos, Janos, Dr., Röntgenstrasse 24, 22335 Hamburg (DE); Medgyesi, György, Röntgenstrasse 24, 22335 Hamburg (DE)
(74) Vertreter: Volmer, Georg, Dipl.-Ing.

(56) Entgegenhaltungen:
- EP-A- 0 033 497
- EP-A- 0 648 465
- DE-U- 9 111 029

## Beschreibung

Die Erfindung bezieht sich auf ein Röntgenuntersuchungsgerät mit einem um eine horizontale Achse schwenkbaren Patientenlagerungstisch, einer in Tischlängsrichtung verfahrbaren Bildwandler-Anordnung und einem darauf ausgerichteten Übertisch-Röntgenstrahler.

Röntgenuntersuchungsgeräte dieser Art sind in der Praxis bekannt, z.B das aus EP-A-0 648 465 bekannte Gerät oder das Gerät "DIAGNOST 120" der Firma Philips. Röntgenstrahler und Bildwandleranordnung befinden sich dabei an einem in Tischlängsrichtung verfahrbaren Wagen. Der Aufbau eines solchen Röntgenuntersuchungsgerätes ist relativ kompliziert und eignet sich nur in begrenztem Maße für unterschiedliche Untersuchungsverfahren. Aufgabe der vorliegenden Erfindung ist es daher, ein Röntgenuntersuchungsgerät der eingangs genannten Art so auszugestalten, daß sich bei einfachem Aufbau universellere Einsatzmöglichkeiten ergeben.

Diese Aufgabe wird erfindungsgemäß dadurch gelöst, daß der Röntgenstrahler an einem horizontal verschiebbaren Stativ in vertikaler Richtung verschiebbar gehaltert und über eine mechanisches Koppelglied so mit dem Patientenlagerungstisch koppelbar ist, daß bei einer Schwenkung des Patientenlagerungstisches das Stativ und der Röntgenstrahler so verschoben werden, daß der Abstand und die Ausrichtung zwischen der Bildwandler-Anordnung und dem Röntgenstrahler unverändert bleibt.

Das erfindungsgemäße Röntgenuntersuchungsgerät umfaßt also neben dem Patientenlagerungstisch mit der in Tischlängsrichtung verfahrbaren Bildwandleranordnung ein Stativ, das horizontal verschiebbar ist und an dem der Röntgenstrahler in vertikaler Richtung verschiebbar gehaltert ist. Durch das Koppelglied können der Patientenlagerungstisch und der Röntgenstrahler so miteinander gekoppelt werden, daß sich bei einer Schwenkung des Patientenlagerungstisches das Stativ horizontal und der Röntgenstrahler vertikal verschiebt, wobei der Abstand und die Ausrichtung zwischen Bildwandler und dem Röntgenstrahler unverändert bleiben. Mit einem solchen Röntgengerät lassen sich also alle gängigen Durchleuchtungsverfahren ausführen. Darüberhinaus kann man mit dem erfindungsgemäßen Röntgenuntersuchungsgerät auch Bucky-Aufnahmen mit vertikalem Strahlengang ausführen. Die Verschiebemöglichkeit des Stativs gestattet die Verwendung eines Patientenlagerungstisches, der keine "schwimmende" Tischplatte aufweist, sondern eine bezüglich des Tischgestells feste Tischplatte, was die Konstruktion deutlich vereinfacht.

Wenn man das Koppelglied vom Röntgenstrahler abkoppelt, ergeben sich weitere Möglichkeiten. Bei der Ausgestaltung nach Anspruch 2 lassen sich Röntgenaufnahmen mit dem Röntgenstrahler auf einem gesonderten Bildaufnehmer, beispielsweise an einem Bucky-Wandstativ ausführen, wobei der Strahlengang horizontal und parallel zur Verschieberichtung des Stativs verlaufen kann. Die Ausgestaltung nach Anspruch 3 erlaubt dabei die Anpassung an unterschiedliche Patientengrößen. Die Weiterbildung nach Anspruch 4 gestattet Röntgenaufnahmen mit horizontal und senkrecht zur Tischlängsrichtung verlaufendem Strahlengang.

Bei der Ausgestaltung nach Anspruch 5 ergeben sich im ersten Betriebsmodus (starre Kopplung zwischen Röntgenstrahler und Bildwandler) die schon erläuterten Untersuchungsmöglichkeiten; im zweiten Betriebsmodus sind lineare Tomographie oder Schrägaufnahmen möglich.

Der erste und der zweite Betriebsmodus können mit der Ausgestaltung nach Anspruch 6 realisiert werden. Wenn der die Einheit zur Schichthöheneinstellung tragende Wagen verfahren wird (erster Betriebsmodus), sind die geschilderten Röntgendurchleuchtungen oder Bucky-Aufnahmen möglich. Wenn hingegen der Wagen bezüglich des Patientenlagerungstisches blockiert ist, ergibt sich eine gegensinnige Verschiebung von Röntgenstrahler und Bildwandleranordnung. Damit sind beispielsweise Schrägaufnahmen möglich. Anspruch 7 beschreibt eine bevorzugte Ausgestaltung der Erfindung, mit deren Hilfe im ersten Betriebsmodus die Lage des Untersuchungsbereichs verändert und im zweiten Betriebsmodus eine lineare Tomographie durchgeführt werden kann.

Anspruch 8 trägt der Tatsache Rechnung, daß die Bildwandleranordnung für eine Röntgendurchleuchtung und für eine Röntgenaufnahme in der Regel getrennte Bildwandler verwendet. Dabei ist es grundsätzlich möglich, für die Einheit zur Schichthöheneinstellung einen zusätzlichen Wagen zu verwenden.

Anspruch 9 beschreibt eine demgegenüber bevorzugte Ausgestaltung, die mechanisch einfacher und stabiler ausgeführt werden kann. Die weitere Ausgestaltung nach Anspruch 10 ermöglicht dabei einen sehr einfachen Übergang zwischen den beiden Betriebsmodi. Wenn die beiden Wagen mit der gleichen Geschwindigkeit verfahren werden (oder beide Wagen stillstehen), sind Röntgendurchleuchtungen oder Bucky-Aufnahmen möglich (erster Betriebsmodus). Wenn die zweite Antriebseinrichtung stillgelegt ist und die erste Antriebseinrichtung auf den mit dem Koppelglied gekoppelten Wagen einwirkt, kann eine lineare Tomographie durchgeführt werden. - Für die beiden Antriebseinrichtungen kann dabei ein gemeinsamer Antriebsmotor verwendet werden, der über eine ein- und ausschaltbare Kupplung auf ein Antriebsglied, z.B. in Form einer Antriebsspindel, einwirkt.

Die Erfindung wird nachstehend anhand der Zeichnungen näher erläutert. Es zeigen:
- Fig. 1: eine Frontansicht einer ersten Ausführungsform,
- Fig. 2: eine Seitenansicht im Bucky-Betrieb,
- Fig. 3: eine Seitenansicht bei einer Durchleuchtung,
- Fig. 4: eine Seitenansicht bei Schräg- oder Schichtaufnahmen,
- Fig. 5: eine Seitenansicht bei horizontalem Strahlengang (in Tischlängsrichtung),
- Fig. 6: eine Seitenansicht bei horizontalem Strahlengang (quer zur Tischlängsrichtung),
- Fig. 7: die Bewegungsmöglichkeiten des Gerätes bei Schichtaufnahmen,
- Fig. 8: einen Teil einer Frontansicht einer verbesserten Ausführungsform,
- Fig. 9: den sich dabei ergebenden Bewegungsbereich bei Schichtaufnahmen und
- Fig. 10: eine Teilansicht einer nochmals verbesserten Ausführungsform.

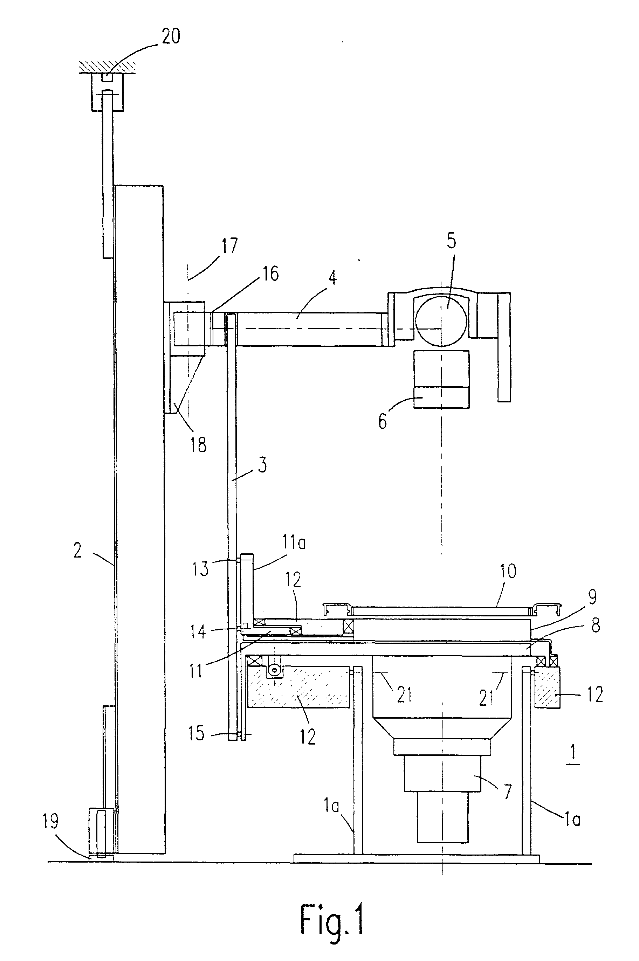

Das in Fig. 1 dargestellte Röntgenuntersuchungsgerät umfaßt drei Hauptkomponenten: Einen Patientenlagerungstisch 1, ein Stativ 2 und ein Koppelglied 3.

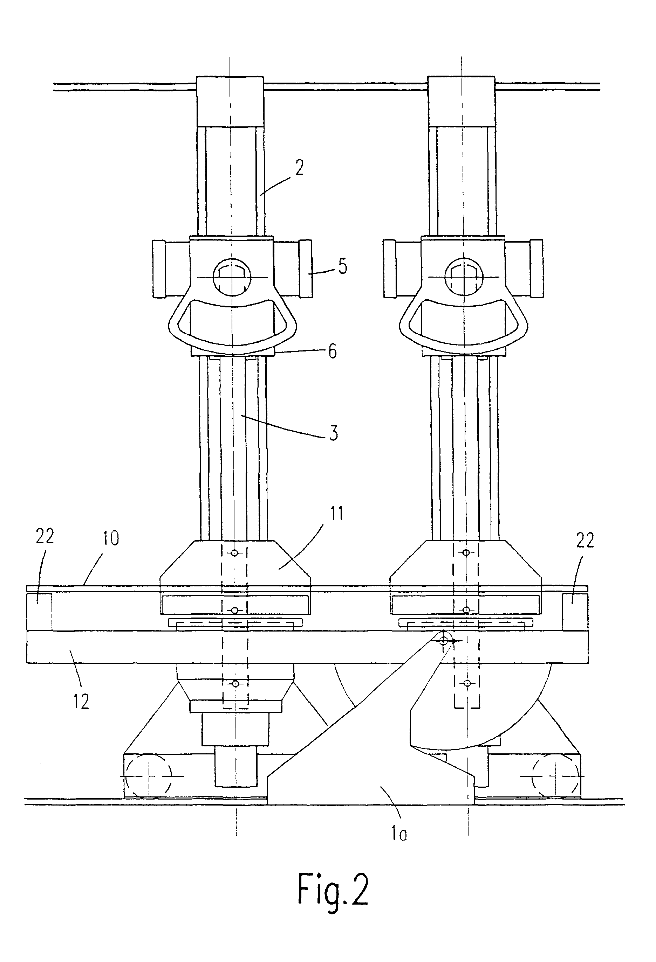

Auf dem Grundgestell 1a des Patientenlagerungstisches 1 sind die Holme 12 eines Tischrahmens um die Achsen 21 drehbar gelagert. Der Rahmen wird durch Verbindungstraversen 22 (Fig. 2) ergänzt, die die Holme miteinander verbinden. Auf den Verbindungstraversen 22 ist eine Tischplatte 10 befestigt. In Längsrichtung der Holme 12 sind drei Wagen verfahrbar: ein Wagen 8 für einen Bildverstärker 7 zur Durchführung von Durchleuchtungen, ein Laufrasterwagen 9 für die Anfertigung von Röntgen- (Bucky) Aufnahmen auf eine darin befindliche Filmkassette und ein Wagen 11 für eine Einheit 11a zur Schichthöheneinstellung, die im folgenden auch als Schichtbock (Fulcrum) bezeichnet wird.

Das Stativ 2 ist auf Schienen 19 und 20 geführt, die sich am Boden und an der Decke (oder einer Wand) befinden und parallel zur Längsrichtung des Tisches (bei horizontaler Tischplatte) verlaufen. An dem Stativ ist ein Wagen 18 vertikal verfahrbar und mit einem nicht näher dargestellten Gewichtsausgleich gekoppelt. Der Wagen 18 trägt an einem Tragarm 4 einen Röntgenstrahler 5 mit einer Tiefenblende 6. Der Tragarm 4 ist mittels eines Drehlagers 16 um seine (horizontale) Längsachse schwenkbar, und das Drehlager 16 seinerseits ist um eine vertikale Achse 17 am Wagen 18 schwenkbar.

Das Koppelglied 3 koppelt den Tragarm 4 und die drei erwähnten Wagen im Patientenlagerungstisch 1 miteinander. Das Koppelglied 3 kann eine Stange sein, jedoch sind auch andere Vorrichtungen denkbar, die eine mechanisch definierte Kopplung zwischen dem Tragarm 4 und den Wagen bewirken. Das Koppelglied 3 ist über ein in der Höhe verstellbares Drehgelenk 13 mit dem Schichtbock 11a verbunden. Außerdem ist es über die Koppelbolzen 14 und 15 mit den Wagen 9 und 8 gekoppelt, so daß diese bei einer Bewegung der Koppelstange relativ zum Patientenlagerungstisch mitgeführt werden. Diese Koppelmittel (11, 11a, 14, 15 und weitere Komponenten) erlauben die Vorgabe zweier unterschiedlicher Betriebsmodi, die anhand der nachfolgenden Figuren erläutert werden.

Fig. 2 zeigt das Röntgenuntersuchungsgerät bei horizontaler Tischplatte 10 in einem die Anfertigung von Bucky-Aufnahmen geeigneten Betriebsmodus, wobei das Stativ in einer ersten und einer zweiten Arbeitsstellung dargestellt ist. Die Verschiebung zwischen diesen beiden Arbeitsstellungen kann mittels eines Spindelantriebs 28 (Fig. 1) erfolgen, mit dessen Hilfe der Wagen 8 und - über das Koppelglied 3 - die Wagen 9 und 11 sowie der Tragarm mit dem Stativ verschoben werden. Anstelle dieses von einem geeigneten Motor angetriebenen Spindelantriebs kann auch ein Kettenantrieb verwendet werden oder eine Zahnstange, die mit einem von einem Motor angetriebenen Zahnrad zusammenwirkt.

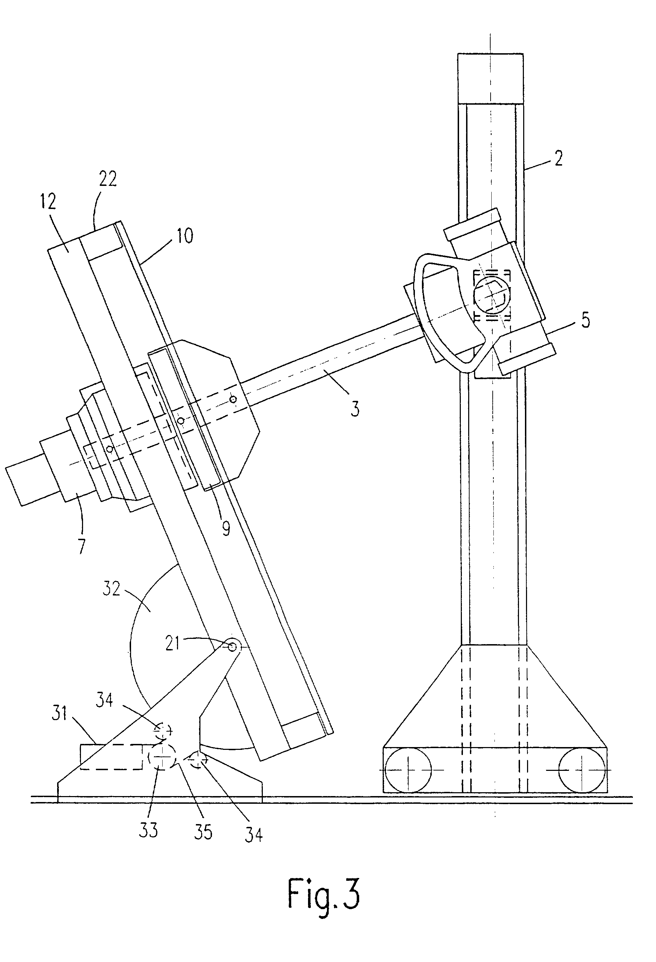

Fig. 3 zeigt das Röntgenuntersuchungsgerät in einer Arbeitsstellung für eine Röntgendurchleuchtung. Der Rahmen 12, 22 und die Tischplatte befinden sich dabei in einer zur Horizontalen geneigten Stellung. Diese Umlegung des Tisches erfolgt mittels eines Getriebemotors 31, der über eine Kette 35, ein Kettenrad 33 und zwei Umlenkrollen 34 ein Segment 32 antreibt, das mit dem Rahmen 12, 22 verbunden ist. Bei einer Schwenkbewegung bewirkt das Koppelglied 3 eine Verschiebung des Stativs nach rechts oder links und gleichzeitig eine Verschiebung des Wagens 18 mit dem Röntgenstrahler 5 nach unten oder nach oben, wobei der Abstand und die Ausrichtung zwischen der Bildwandleranordnung 7 (bzw. 9) und dem Röntgenstrahler 5 unverändert bleiben. Auch wenn der Wagen 8 und (mit ihm die Wagen 9 und 11) in Tischlängsrichtung verfahren werden, wird die Röntgenröhre über das Koppelglied 3 so mitgeführt, daß der Abstand und die Ausrichtung zwischen der Bildwandleranordnung 7 (bzw. 9) und dem Röntgenstrahler 5 unverändert bleiben.

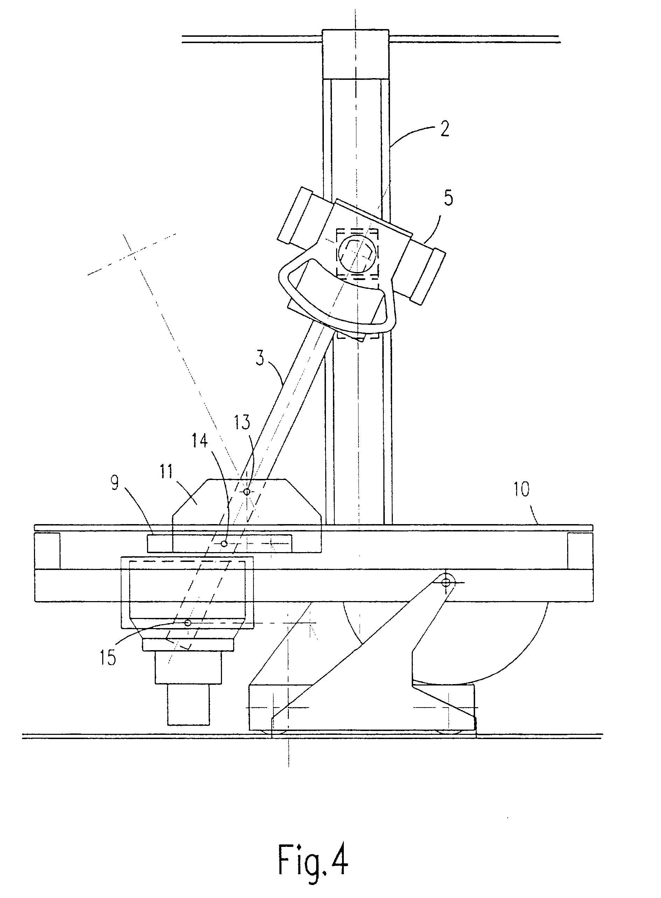

Fig. 4 zeigt das Röntgenuntersuchungsgerät bei horizontaler Tischplatte in einem anderen Betriebsmodus. Dieser Betriebsmodus ergibt sich dann, wenn der Wagen 11 (z.B. durch eine Bremse oder eine formschlüssige Arretierung an dem oberen Holm 12) festgesetzt wird und damit bezüglich des Patientenlagerungstisches blockiert ist. Wird dann der Wagen 8 mit dem Bildverstärker verschoben, wird die Koppelstange (über dem Zapfen 15) mitgenommen, wobei das Koppelglied 3 um den jetzt feststehenden (ortsfesten) Drehpunkt 15 geschwenkt wird, wodurch das Stativ 2 seitlich verschoben wird. Der Laufrasterwagen 9 wird über den Zapfen 14 mitgeführt, so daß sich eine Schrägprojektion ergibt, bei der der Zentralstrahl stets auf die Mitte einer in dem Laufrasterwagen 9 befindlichen Filmkassette ausgerichtet ist.

Wenn der Laufrasterwagen 9 mittels des Antiebs 28 kontinuierlich so verfahren wird, daß das Koppelglied 3 von seiner dargestellten Position in die gestrichelte Position geschwenkt wird, kann während dieser Schwenkbewegung eine Schichtaufnahme mit einer linearen Verwischungsbewegung (lineare Tomographie) ausgeführt werden. Verschiebt man die Höhe des Drehgelenkes bzw. Koppelpunktes 13, ändert sich die Höhe der bei einer Schichtaufnahme scharf abgebildeten Schicht entsprechend. Wenn der Koppelpunkt 13 genügend weit nach unten verschoben wird, sind auch Schrägdurchleuchtungen möglich, bei denen der Zentralstrahl im Zentrum des Bildverstärkers 7 auftrifft.

Während bei dem in Fig. 4 dargestellten zweiten Betriebsmodus der Wagen am Tisch 1 fixiert ist, sind bei dem ersten Betriebsmodus nach Fig. 2 oder 3 die drei Wagen 8, 9, 11 starr miteinander gekoppelt, so daß bei einer Verschiebung des Wagens 8 auch die Wagen 9 und 11 ebenfalls verschoben werden, wobei sie über das Koppelglied 3 das Stativ 2 mitnehmen.

Durch Abkoppeln des Koppelgliedes 3 von dem Tragarm 4 ergeben sich weitere Möglichkeiten:

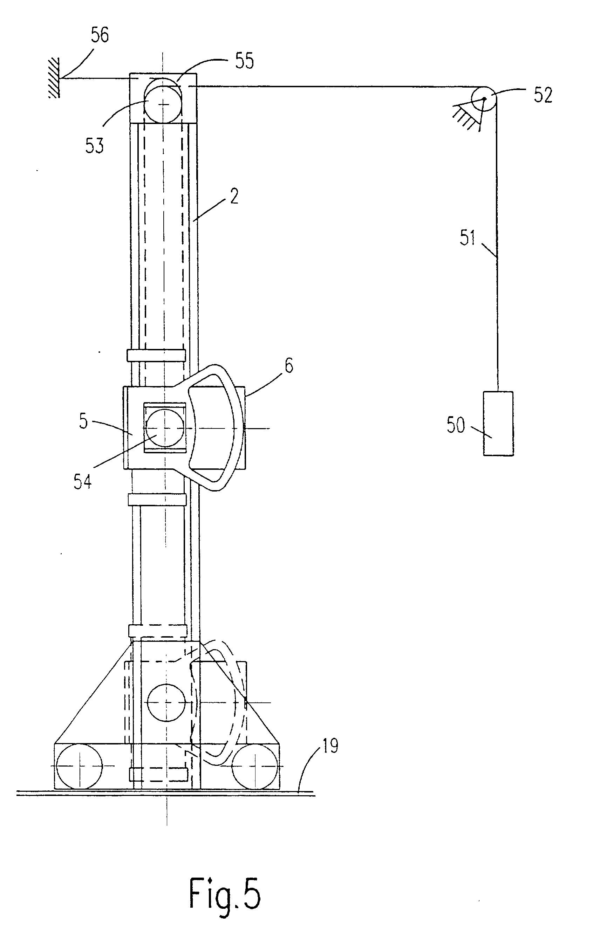

Das Stativ 2 kann - auch an dem Patientenlagerungstisch vorbei - verschoben werden, wie in Fig. 5 dargestellt, und der Röntgenstrahler 5 kann in dem Drehlager 16 (vergl. Fig. 1) um 90° geschwenkt werden, so daß seitliche Aufnahmen auf ein nicht näher dargestelltes Wandstativ möglich sind. Mit Hilfe des Wagens 18 (Fig. 1) kann der Röntgenstrahler 5 dabei vertikal in beliebige Positionen verschoben werden, wie in Fig. 5 gestrichelt angedeutet.

Fig. 5 stellt darüberhinaus den schon erwähnten Gewichtsausgleich schematisch dar. Er umfaßt ein Gegengewicht 50, das das Gewicht des Wagens 18 mitsamt dem Tragarm 4 und des Röntgenstrahlers 5 ausgleicht. Wenn das Gegengewicht 50 innerhalb des Stativs gegensinnig zum Röntgenstrahler 5 bewegbar wäre, müßte es bei einer Verschiebung des Stativs mitbewegt werden. Das Gegengewicht ist jedoch durch ein Seil 51, das über eine (ortsfeste) Rolle 52 an der Wand oder Decke, eine erste Rolle 53 am Stativ 2, eine Rolle 54 am Wagen 18 und eine zweite Rolle 55 am Stativ 2 geführt ist, mit einem ortsfesten Punkt 56 verbunden. Das Gegengewicht kann bei diese Rollenanordnung seine Lage im Raum nur in vertikaler Richtung, aber nicht in horizontaler Richtung ändern. Das hat den Vorteil, daß es bei einer Verschiebung des Stativs 2 nicht mitbewegt- und beschleunigt werden muß.

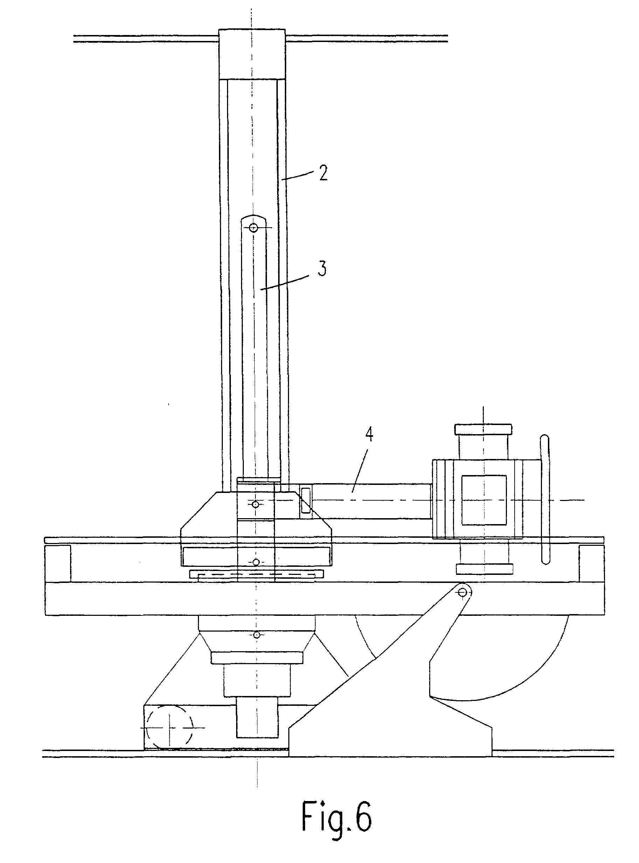

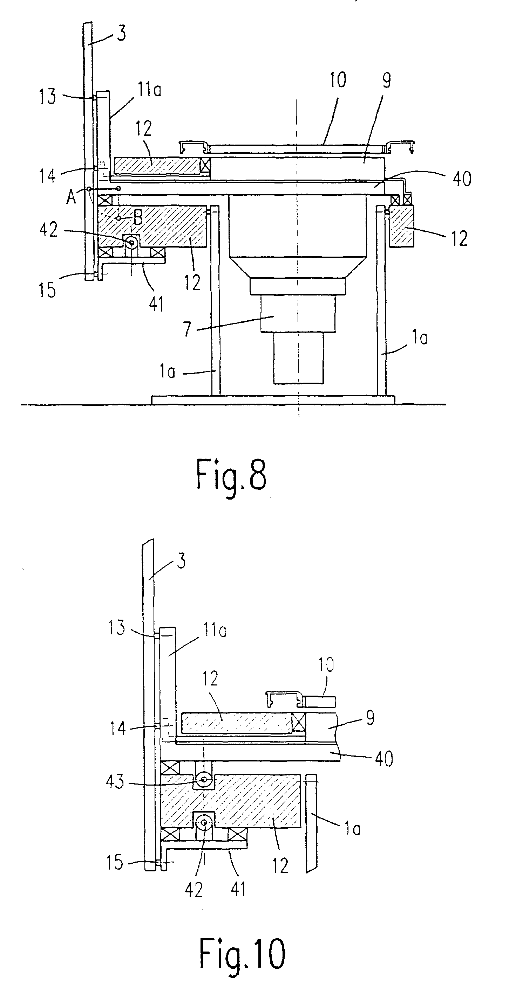

Fig. 6 zeigt eine weitere Aufnahmemöglichkeit in entkoppeltem Zustand. Diese ergibt sich dann, wenn der Tragarm 4 um die vertikale Achse 17 geschwenkt und dann um 90° um die horizontale Achse gedreht wird. Es ergibt sich dann ein horizontaler, zur Tischlängsrichtung senkrechter Strahlengang.

Fig. 8 zeigt eine verbesserte Ausführungsform der Erfindung in einer Frontansicht, wobei im wesentlichen nur der Patientenlagerungstisch dargestellt ist.

Während bei der Ausführungsform nach Fig. 1 der Schichtbock 11a von dem Wagen 11 und der Bildverstärker 7 von dem gesonderten Wagen 8 getragen wird, ist bei der Ausführungsform nach Fig. 8 für den Schichtbock 11a und dem Bildverstärker 7 ein gemeinsamer Wagen 40 vorgesehen, der eine stabilere Führung des Schichtbockes gewährleistet. Für die Schwenkung bzw. für die Verschiebung des Koppelgliedes 3 ist auf der Unterseite des linken unteren Holms 12 ein Antriebswagen 41 vorgesehen, der über den Koppelbolzen 15 auf das untere Ende des Koppelgliedes einwirkt und der linear in Tischlängsrichtung geführt ist. Auf den Antriebswagen 41 wirkt ein Spindelantrieb 42 (oder ein Kettenantrieb oder dergleichen) ein, wobei das Verhalten davon abhängt, ob der Wagen 40 mit dem Koppelglied 3 gekoppelt ist (fett ausgezogene Verbindung zu Punkt A) oder mit dem Holm 12 gekoppelt ist (dünn dargestellte Kopplung mit dem Punkt B am Holm 12), so daß der Wagen 40 nicht verschoben werden kann.

Bei einer Kopplung des Wagens 40 mit dem Koppelglied 3 (am Punkt A) werden der Wagen 40. das Koppelglied 3 und der Wagen 9 gleichsinnig ( in der gleichen Richtung und um die gleiche Strecke) verschoben entsprechend Fig. 2 oder Fig 3 (erster Betriebsmodus). - Ist der Wagen 40 mit dem Punkt B gekoppelt, also bezüglich des Holms 12 fixiert, schwenkt der Antriebswagen über den Koppelbolzen 15 das Koppelglied 3 um das Gelenk 13 entsprechend Fig. 4. Von Vorteil ist dabei, daß der Bildverstärker 7 nicht mitbewegt wird (der Wagen 40 steht dann still), so daß der Antrieb 42 dafür keine Beschleunigungs- oder Bremskräfte aufbringen muß. Der Bildverstärker 7 wird ja für Schichtaufnahmen nicht benötigt.

Ein weiterer Vorteil ist der vergrößerte Bereich, innerhalb dessen Schichtaufnahmen ausgeführt werden können.

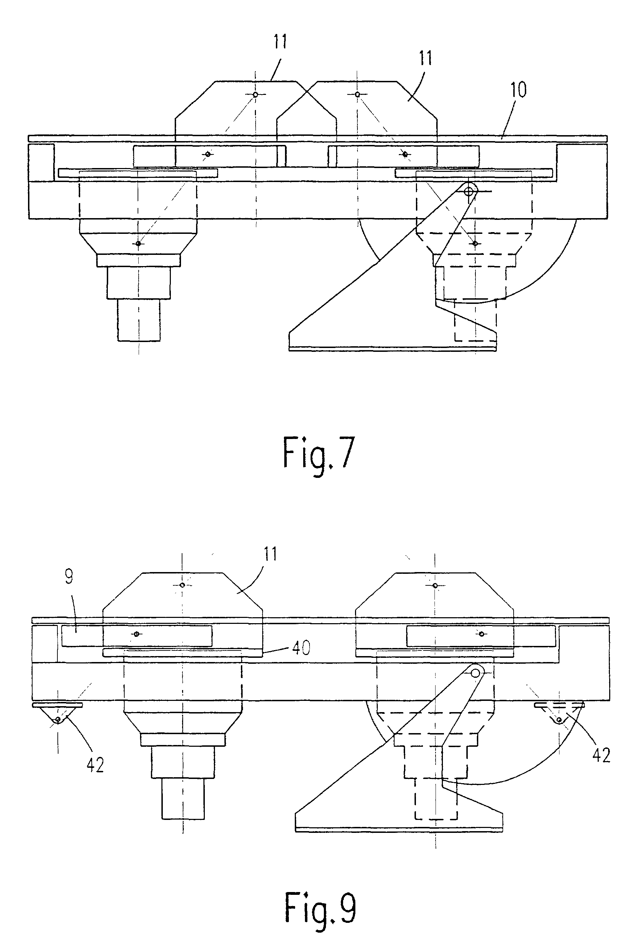

Fig. 7 zeigt die Verhältnisse bei dem Gerät nach Fig. 1 in schematischer Darstellung. Man erkennt, daß der Schichtbock 11a für Schichtaufnahmen kopfoder fußseitig nur in die in Fig. 7 dargestellten Positionen gebracht werden kann, wenn eine Kollision des Bildverstärkers 7 bzw. des ihn tragenden Wagens mit den Verbindungstraversen 22 am kopf- und fußseitigen Ende des Patientenlagerungstisches vermieden werden soll. Bereiche, die weiter am Kopf- oder Fußende liegen, sind einer Schichtaufnahme nicht zugänglich, was insbesondere dann von Nachteil ist, wenn - wie vorausgesetzt - die Tischplatte 10 bezüglich des Rahmens nicht verschoben werden kann.

Fig. 9 zeigt demgegenüber die Verhältnisse bei der Ausführungsform nach Fig. 8. Man erkennt, daß die kopf- und fußseitigen Endpositionen deutlich weiter auseinander liegen können als bei der Fig. 7, weil der voluminöse Bildverstärker 7 und der ihn tragende Wagen 40 dabei nicht bewegt werden, sondern nur der relativ schmale Antriebswagen 42, der unterhalb des linken unteren Holmes geführt ist. Dadurch wird der Schichtaufnahme abbildbare Bereich eines Patienten wesentlich vergrößert.

Fig. 10 zeigt eine Verbesserung der Ausführungsform nach Fig. 8. Diese Verbesserung besteht darin, daß der Wagen 40 von einem Spindelantrieb 43 (oder einem Kettenantrieb oder eine Zahnstange) verschoben werden kann. Dabei sind folgende Betriebsweisen möglich:

Im ersten Betriebsmodus sind sowohl der Antrieb 41 für den Wagen 42 als auch der Antrieb 43 für den Wagen 40 wirksam, und zwar so, daß die beiden Wagen 40 und 42 mit der gleichen Geschwindigkeit in Kopf- oder Fußrichtung des Tisches verschoben werden. Das Koppelglied 3 verschiebt sich dabei lateral und nimmt das Stativ mit. In diesem Betriebsmodus können Bucky-Aufnahmen entsprechend Fig. 2 oder Röntgendurchleuchtungen gemäß Fig. 3 durchgeführt werden, bei denen der Röntgenstrahler parallel zur jeweiligen Tischlängsrichtung verschoben wird.

In dem zweiten Betriebsmodus ist der Spindelantrieb 43 eingeschaltet (d.h. der Wagen 40 kann sich nicht bezüglich des Holmes 12 verschieben, während der Spindelantrieb 41 auf den Wagen 42 einwirken kann. Dadurch wird das Koppelglied 3 und das Drehgelenk 13 geschwenkt, und es ergibt sich eine Schichtaufnahme gemäß Fig. 4 bzw. Fig. 9.

Der Vorteil dieser Ausführungsform ist, daß es nicht erforderlich ist, die verschiedenen Wagen bzw. das Koppelglied 3 je nach Betriebsmodus miteinander zu verriegeln, wie bei den Ausführungsformen nach Fig. 1 bzw. Fig. 8. Außerdem ist ein Umschalten zwischen den beiden Betriebsmodi leicht mittels an geeigneter Stelle angebrachter Schalter möglich. Im Prinzip kann für beide Spindelantriebe 41 und 43 ein gemeinsamer Antriebsmotor vorgesehen sein, der über eine elektrisch ein- und ausschaltbare Kupplung auf den Spindelantrieb 43 einwirkt, da entweder beide Antriebe synchron laufen (erster Betriebsmodus) oder nur der Antrieb 41 (zweiter Betriebsmodus).

## Patentansprüche

1. Röntgenuntersuchungsgerät mit einem um eine horizontale Achse (21) schwenkbaren Patientenlagerungstisch (1), einer in Tischlängsrichtung verfahrbaren Bildwandler-Anordnung (7, 9) und einem darauf ausgerichteten Übertisch-Röntgenstrahler (5),
**dadurch gekennzeichnet, daß** der Röntgenstrahler (5) an einem horizontal verschiebbaren Stativ (2) in vertikaler Richtung verschiebbar gehaltert und über eine mechanisches Koppelglied (3) so mit dem Patientenlagerungstisch koppelbar ist, daß bei einer Schwenkung des Patientenlagerungstisches (1) das Stativ (2) und der Röntgenstrahler (5) so verschoben werden, daß der Abstand und die Ausrichtung zwischen der Bildwandler-Anordnung (7,9) und dem Röntgenstrahler (5) unverändert bleibt.

2. Röntgenuntersuchungsgerät nach Anspruch 1,
**dadurch gekennzeichnet, daß** der Röntgenstrahler (5) an einem horizontalen, um seine Mittelachse schwenkbaren Arm befestigt ist (4), der lösbar mit dem Koppelglied (3) verbunden ist.

3. Röntgenuntersuchungsgerät nach Anspruch 2,
**dadurch gekennzeichnet, daß** der Arm (4) an einem vertikal am Stativ (2) verfahrbaren Wagen (18) befestigt ist.

4. Röntgenuntersuchungsgerät nach Anspruch 3,
**dadurch gekennzeichnet, daß** der Arm (4) um eine vertikale Achse (17) schwenkbar an dem Wagen (18) befestigt ist.

5. Röntgenuntersuchungsgerät nach Anspruch 1,
**dadurch gekennzeichnet, daß** Koppelmittel (11, 11a, 14, 15 ; A, B; 42, 43) vorgesehen sind, durch die das Koppelglied (3) in einem ersten Betriebsmodus eine starre Kopplung zwischen dem Röntgenstrahler (5) und der Bildwandler-Anordnung (7, 9) bewirkt und durch die das Koppelglied (3) in einem zweiten Betriebsmodus eine gegensinnige Verschiebung zwischen dem Röntgenstrahler (5) und wenigstens einem Teil (9) der Bildwandler-Anordnung (7, 9) bewirkt.

6. Röntgenuntersuchungsgerät nach Anspruch 5,
**dadurch gekennzeichnet, daß** die Koppelmittel (3) eine an einem in Längsrichtung des Patientenlagrungstisches (1) verfahrbaren Wagen (11) befestigte Einheit (11a) zur Schichthöheneinstellung umfassen, daß das Koppelglied (3) in einem in der Höhe verstellbaren Koppelpunkt (15) an Einheit (11a) zur Schichthöheneinstellung angelenkt ist, und daß der Wagen (11) in dem ersten Betriebsmodus frei verfahrbar und im zweiten Betriebsmodus bezüglich des Patientenlagerungstisches (1) blockiert ist.

7. Röntgenuntersuchungsgerät nach Anspruch 6,
**dadurch gekennzeichnet, daß** der Patientenlagerungstisch (1) eine auf einen in Längsrichtung des Patientenlagerungstisches verfahrbaren Wagen (8) einwirkende Antriebseinrichtung (28) aufweist, und daß das Koppelglied (3) im Abstand von dem Koppelpunkt (15) mit diesem Wagen (8) gekoppelt ist.

8. Röntgenuntersuchungsgerät nach Anspruch 5,
**dadurch gekennzeichnet, daß** die Bildwandler-Anordnung einen Durchleuchtungs-Bildwandler (7) und einen Aufnahme-Bildwandler (9) umfaßt, die auf je einem mit dem Koppelglied (3) gekoppelten, in Längsrichtung des Patientenlagerungstisches verfahrbaren Wagen (8, 9) angeordnet sind.

9. Röntgenuntersuchungsgerät nach Anspruch 6 und 8,
**dadurch gekennzeichnet, daß** der Durchleuchtungs-Bildwandler (7) und die Einheit (11a) zur Schichthöheneinstellung auf einem gemeinsamen Wagen (40) angeordnet sind, daß der Patientenlagerungstisch (10) eine auf einen in Längsrichtung des Patientenlagerungstisches verfahrbaren Wagen (41) einwirkende erste Antriebseinrichtung (42) aufweist, daß das Koppelglied (3) im Abstand von dem Koppelpunkt (15) mit diesem Wagen gekoppelt ist, und daß die Koppelmittel (3,11a, A, B) im ersten Betriebsmodus die beiden Wagen (40,41) miteinander koppeln und im zweiten Betriebsmodus den Wagen (40) mit dem Durchleuchtungs-Bildwandler (7) bezüglich des Patientenlagerungstisches (10) blockieren.

10. Röntgenuntersuchungsgerät nach Anspruch 9,
**dadurch gekennzeichnet, daß** eine zweite Antriebseinrichtung (43) vorgesehen ist, die auf den Wagen (40) mit dem Durchleuchtungs-Bildwandler einwirkt, und daß die beiden Antriebseinrichtung (42, 43) so gesteuert sind, daß im ersten Betriebsmodus die beiden Wagen (40, 41) mit der gleichen Geschwindigkeit und in der gleichen Richtung verfahren werden und im zweiten Betriebsmodus die zweite Antriebseinrichtung (43) stillgelegt ist.

## Claims

1. An X-ray examination apparatus which includes a patient table (1) which is tiltable about a horizontal axis (21), an image converter arrangement (7, 9) which is displaceable in the longitudinal direction of the table, and an overtable X-ray source (5) which is aligned with respect thereto, **characterized in that** the X-ray source (5) is mounted on a horizontally displaceable stand (2) so as to be displaceable in the vertical direction and can be coupled, via a mechanical coupling member (3), to the patient table in such a manner that upon tilting of the patient table (1) the stand (2) and the X-ray source (5) are displaced in such a manner that the distance between and the alignment of the image converter arrangement (7, 9) and the X-ray source (5) with respect to one another remain the same.

2. An X-ray examination apparatus as claimed in Claim 1, **characterized in that** the X-ray source (5) is mounted on a horizontal arm (4) which is tiltable about its central axis and is detachably connected to the coupling member (3).

3. An X-ray examination apparatus as claimed in Claim 2, **characterized in that** the arm (4) is connected to a carriage (18) which is displaceable in the vertical direction on the stand (2).

4. An X-ray examination apparatus as claimed in Claim 3, **characterized in that** the arm (4) is connected to the carriage (18) so as to be tiltable about a vertical axis (17).

5. An X-ray examination apparatus as claimed in Claim 1, **characterized in that** there are provided coupling means (11, 11a, 14, 15; A, B; 42, 43) whereby the coupling member (3) establishes a rigid coupling between the X-ray source (5) and the image converter arrangement (7, 9) in a first mode of operation and whereby the coupling member (3) provides a displacement in opposite directions between the X-ray source (5) and at least a part (9) of the image converter arrangement (7, 9) in a second mode of operation.

6. An X-ray examination apparatus as claimed in Claim 5, **characterized in that** the coupling member (3) includes a unit (11a) for slice height adjustment which is coupled to a carriage (11) which is displaceable in the longitudinal direction of the patient table (1), that the coupling member (3) is hinged to the unit (11a) for adjusting the slice height at a coupling point (15) whose height can be adjusted, and that the carriage (11) can be displaced freely in the first mode of operation and is blocked relative to the patient table (1) in the second mode of operation.

7. An X-ray examination apparatus as claimed in Claim 6, **characterized in that** the patient table (1) includes a drive device (28) which acts on a carriage (8) which is displaceable in the longitudinal direction of the patient table, and that the coupling member (3) is coupled to this carriage (8) at a distance from the coupling point (15).

8. An X-ray examination apparatus as claimed in Claim 5, **characterized in that** the image converter arrangement includes a fluoroscopy image converter (7) and an exposure image converter (9) which are mounted on a respective carriage (8, 9) which is coupled to the coupling member (3) and is displaceable in the longitudinal direction of the patient table.

9. An X-ray examination apparatus as claimed in the Claims 6 and 8, **characterized in that** the fluoroscopy image converter (7) and the unit (11a) for slice height adjustment are accommodated on a common carriage (40), that the patient table (10) includes a first drive device (42) which acts on a carriage (41) which is displaceable in the longitudinal direction of the patient table, that the coupling member (3) is coupled to this carriage at a distance from the coupling point (15), and that the coupling means (3, 11a, A, B) couple the two carriages (40, 41) to one another in the first mode of operation and block the carriage (40) with the fluoroscopy image converter (7) relative to the patient table (10) in the second mode of operation.

10. An X-ray examination apparatus as claimed in Claim 9, **characterized in that** there is provided a second drive device (43) which acts on the carriage (40) with the fluoroscopy image converter, and that the two drive devices (42, 43) are controlled in such a manner that the two carriages (40, 41) are displaced at the same speed and in the same direction in the first mode of operation and that the second drive device (42) is deactivated in the second mode of operation.

## Revendications

1. Installation d'examen par rayons X munie d'un support pour patient (1) pivotable autour d'un axe horizontal, un dispositif convertisseur d'image (7, 9) déplaçable dans la direction longitudinale du support et un générateur de rayons X (5) disposé au-dessus du support,
**caractérisée en ce que** le générateur de rayons X (5) est logé à déplacement en direction verticale sur un trépied (2) coulissable horizontalement et peut être couplé par l'intermédiaire d'un organe de couplage mécanique (3) avec le support pour patient de telle sorte qu'en cas de pivotement du support pour patient (1), le trépied (2) et le générateur de rayons X soient déplacés de telle sorte que l'intervalle et l'orientation entre le dispositif convertisseur d'image (7, 9) et le générateur pour rayons X (5) restent inchangés.

2. Installation d'examen par rayons X selon la revendication 1,
**caractérisée en ce que** le générateur de rayons X (5) est fixé à un bras (4) pivotant autour de son axe central qui est relié de manière amovible à l'organe de couplage (5).

3. Installation d'examen par rayons X selon la revendication 2,
**caractérisée en ce que** le bras (4) est fixé à un chariot (18) déplaçable verticalement sur le trépied (2).

4. Installation d'examen par rayons X selon la revendication 3,
**caractérisée en ce que** le bras (4) est fixé sur le chariot (18) à pivotement autour d'un axe vertical (17).

5. Installation d'examen par rayons X selon la revendication 1,
**caractérisée en ce qu'**il est prévu des moyens de couplage (11, 11a, 14, 15; A, B; 42, 43) par lesquels l'organe de couplage (3) assure dans un premier mode de fonctionnement un couplage rigide entre le générateur de rayons X (5) et le dispositif convertisseur d'image (7, 9) et par lequel l'organe de couplage (3) assure dans un deuxième mode de fonctionnement un décalage en sens contraire entre le générateur de rayons X (5) et au moins une partie (9) du dispositif convertisseur d'image (7, 9).

6. Installation d'examen par rayons X selon la revendication 5,
**caractérisée en ce, que** les moyens de couplage (3) comprennent une unité (11a) fixée sur un chariot (11) déplaçable dans la direction longitudinale du support pour patient (1) en vue du réglage de la hauteur de couche, que l'organe de couplage (3) est articulé dans un point de couplage (15) déplaçable en hauteur sur l'unité (11a) pour le réglage de la hauteur de couche et que le chariot (11) est déplaçable librement dans le premier mode de fonctionnement et bloqué dans le deuxième mode de fonctionnement par rapport au support pour patient (1).

7. Installation d'examen par rayons X selon la revendication 6,
**caractérisée en ce que** le support pour patient (1) présente un dispositif d'entraînement (28) agissant sur un chariot (8) déplaçable en direction longitudinale du support pour patient et que l'organe de couplage (2) est couplé à une distance du point de couplage (15) avec ce chariot (8).

8. Installation d'examen par rayons X selon la revendication 5,
**caractérisée en ce que** le dispositif convertisseur d'image comprend un convertisseur de radioscopie (7) et un convertisseur de cliché (9) qui sont respectivement disposés sur un chariot (8,9) couplé à l'organe de couplage (3) et déplaçable dans la direction longitudinale du support pour patient.

9. Installation d'examen par rayons X selon l'une des revendications 6 et 8,
**caractérisée en ce que** le convertisseur de radioscopie (7) et l'unité (11a) pour le réglage de la hauteur de couche sont disposés sur un chariot commun (40), que le support pour patient (10) présente un premier dispositif d'entraînement (42) agissant sur un chariot (41) dans la direction longitudinale du support pour patient, que l'organe de couplage (3) est couplé à un intervalle de ce point de couplage (15) avec ce chariot et que les moyens de couplage (3, 11a, A, B) couplent entre eux les deux chariots (40, 41) dans le premier mode de fonctionnement et bloquent le chariot (40) avec le convertisseur d'images de radioscopie (7) par rapport au support du patient (10) dans le deuxième mode de fonctionnement.

10. Installation d'examen pour rayons X selon la revendication 9,
**caractérisée en ce qu'**il est prévu un deuxième dispositif d'entraînement (43), qui agit sur le chariot (40) avec le convertisseur d'images de radioscopie et que les deux dispositifs d'entraînement (42, 43) sont commandés de telle sorte que, dans le premier mode de fonctionnement, les deux chariots (40, 41) soient déplacés à la même vitesse et dans la même direction et, dans le deuxième mode de fonctionnement, le deuxième dispositif d'entraînement (43) soit arrêté.
